# NEW EUROPEAN PATENT SPECIFICATION

(11) **EP 0 836 462 B2**
(45) Date of publication and mention of the opposition decision: **15.09.2004**
(45) Mention of the grant of the patent: 25.10.2000
(21) Application number: 96921573.0
(22) Date of filing: 12.06.1996
(51) Int. Cl.: A61F 13/15

(54) **ABSORBENT ARTICLE WITH EXTENSIBLE AND ARTICULATING PORTIONS**
SAUFÄHIGER ARTIKEL MIT DEHNBAREN UND GELENKARTIGEN TEILEN
ARTICLE ABSORBANT A PARTIES EXTENSIBLES ET ARTICULEES

(30) Priority: 03.07.1995 US 497957
(43) Date of publication of application: 22.04.1998
(73) Proprietor: THE PROCTER & GAMBLE COMPANY, Cincinnati, Ohio 45202 (US)
(72) Inventor: LAVASH, Bruce, William, D-61350 Bad Homburg (DE); OSBORN, Thomas, Ward, III, Cincinnati, OH 45224 (US); MANSFIELD, Michele, Ann, Cincinnati, OH 45220 (US)
(74) Representative: Veronese, Pancrazio
(86) International application number: PCT/US1996/010195
(87) International publication number: WO 1997/001996

(56) References cited:
- WO-A-94/02094
- WO-A-95/03765
- WO-A-96/05789
- WO-A-96/13237
- US-A- 3 776 233
- US-A- 4 950 262

## Description

### FIELD OF THE INVENTION

The present invention relates to absorbent articles such as sanitary napkins, pantiliners, and incontinence pads. More particularly, the present invention relates to absorbent articles that have portions that are able to move and articulate about flexible and extensible portions.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body to prevent body and clothing soiling. Sanitary napkins are a type of absorbent article worn by women in a pair of panties that is normally positioned between the wearer's legs, adjacent to the perineal area of the body.

A wide variety of different types of such absorbent articles are known and described in the art Examples of commercially successful sanitary napkins are described in U.S. Patents 4,950,264 and 5,009,653 issued to Osborn on August 21, 1990, and April 23, 1991, respectively. The search for improved absorbent articles has, however, continued. For example, recent efforts have been directed to providing sanitary napkins that more closely conform to the wearer's body. Other efforts have been directed to providing sanitary napkins with extensibility for this purpose and for improved comfort.

One effort to provide a sanitary napkin that conforms closely to the wearer's body is described in PCT International Publication No. WO 92/10984 entitled "Sanitary Napkin Having Transversely Segmented Core", published in the name of Osborn, et al. on July 9, 1992. Another example which contains a similar disclosure is described in EPO Patent Publication No. 0 605 017 A2, published in the name of Tong-ho J. Hsieh on July 6, 1994. Examples of extensible and stretchable sanitary napkins are described in PCT International Publication No. WO 93/01785 entitled "Stretchable Absorbent Articles", published in the name of Osborn, et al. on February 4, 1993.

It is believed, however, that manufacturing fully extensible or stretchable absorbent articles, such as catamenial products, on a large scale in a high speed operation may be fairly difficult and expensive. The difficulties and expenses arise from the fact that making an entire absorbent article extensible involves the use of more expensive extensible materials as the components of the article and the fact that these materials will stretch, oftentimes in different amounts relative to each other, when being assembled on a high speed manufacturing line.

Thus, a need exists for an absorbent article, such as a sanitary napkin, that is provided with an improved structure that conforms closely to the wearer's body and is less expensive and complex to manufacture than a fully extensible sanitary napkin. In particular, a need exists for a sanitary napkin that can be easily and inexpensively assembled on a high speed manufacturing line, and which is provided with an amount of extensibility that is sufficient to provide the sanitary napkin with improved comfort.

It is, therefore, an object of the present invention to provide an absorbent article, such as a sanitary napkin, that has an improved structure that conforms closely to the wearer's body.

It is another object of the present invention to provide an absorbent article, such as a sanitary napkin, which is provided with an amount of extensibility that is sufficient to provide the sanitary napkin with improved comfort and performance.

It is still another object of the present invention to provide an absorbent article, such as a sanitary napkin, which achieves the objects described above and which is less expensive and complex to manufacture on a high speed manufacturing line than a fully extensible sanitary napkin.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin, pantiliner, or incontinence pad (an 'absorbent pad") that has portions that are able to move and articulate about flexible and extensible portions, said absorbent article having the features as provided in claim 1.

The absorbent article comprises a liquid pervious topsheet, a liquid impervious backsheet joined to the topsheet, and an absorbent core positioned between the topsheet and the backsheet. The absorbent article comprises at least two portions or regions ("expandable portions") that can expand more than other regions of the absorbent article. The expandable portion s of the absorbent article can comprise a portion or region of the topsheet, the backsheet, or both. Preferably, the topsheet, backsheet, the absorbent core, and any other components of the absorbent article are extensible in the expandable portions of the absorbent article so that the absorbent article is extensible through its full thickness in the expandable regions. The expandable portions of the absorbent article can be located in any desired place on the absorbent article, such as along a portion of the perimeter of the absorbent articte. The expandable portions can be extensible in the longitudinal direction, the transverse direction, some direction between the longitudinal and transverse directions, or in more than one of these directions. The expandable portions can all be expandable in the same direction, or they can be expandable in different directions.

In a version of this embodiment, the absorbent article comprises a sanitary napkin having a first portion, a second portion, and a third portion with a first flexible and extensible intermediate portion between the first and second portions, and a second flexible and extensible intermediate portion between the second and third portions. The sanitary napkin is preferably provided with absorbent component structures that are extensible and stretchable (i.e., can retract). The sanitary napkin is preferably also provided with an overall extensibility and stretchability.

Numerous alternative embodiments that employ the features of the present invention are also described. For example, all portions of the expandable regions need not be extensible in the same amount. In addition, the various embodiments described herein can have an overall extensibility that ranges from that of current commercially-available sanitary napkins (for those embodiments which have expandable regions but not an overall extensibility) to that of fully extensible sanitary napkins.

### BRIEF DESCRIPTION OF THE DRAWINGS

While the specification concludes with claims particularly pointing out and distinctly claiming the subject matter which is regarded as forming the present invention, it is believed that the invention will be better understood from the following description when taken in conjunction with the accompanying drawings in which:

FIG. 1 is a simplified, partially cut away top plan view of the sanitary napkin of the present invention which has three relatively inextensible portions that are joined by a pair of flexible and extensible intermediate portions.

FIG. 2 is a side view of the sanitary napkin shown in FIG. 1.

FIG. 3 is a top plan view of the sanitary napkin shown in FIG. 1 shown in an extended condition.

FIG. 4 is a side view of the sanitary napkin shown in FIG. 1 in one possible articulated configuration.

FIG. 5 is a top plan view of an alternative embodiment of the sanitary napkin shown in FIG. 1 that is additionally provided with flexible and laterally extensible portions in each end region of the sanitary napkin.

FIG. 6 is a top plan view of the sanitary napkin shown in FIG. 5 in an extended condition.

FIG. 7 is a top plan view of a web material having a strainable network which is used in the flexible and extensible intermediate portions of the sanitary napkin shown in FIGS. 1-6.

FIGS. 7A-C are enlarged segmented perspective illustrations of the web material shown in FIG. 7 in which the web material moves from an untensioned condition to progressively greater tensioned conditions

FIG. 8 is a schematic side cross-sectional view of a sanitary napkin according to the present invention that has a segmented absorbent core.

FIG. 9 is a schematic side cross-sectional view of the sanitary napkin shown in FIG. 8 in an extended condition.

FIG. 10 is a schematic side cross-sectional view of a sanitary napkin having an alternative construction in which a layer is placed between the absorbent layer and the core segments to reduce interference between such components when they move relative to each other.

FIG. 11 is a schematic side cross-sectional view of the sanitary napkin shown in FIG. 10 in an extended condition.

FIG. 12 is a top plan view of the absorbent components and backsheet of a sanitary napkin having an alternative arrangement of absorbent elements, one of which is partially cut away to show the underlying absorbent core.

FIG. 13 is a top plan view of the absorbent components and backsheet of a sanitary napkin having another alternative arrangement of absorbent elements also having one of the absorbent components partially cut away to show the underlying absorbent core.

FIG. 14 is a bottom plan view of a sanitary napkin which may use the arrangement of absorbent elements shown in FIG. 13, which shows one preterred adhesive fastener configuration

### DETAILED DESCRIPTION OF THE INVENTION

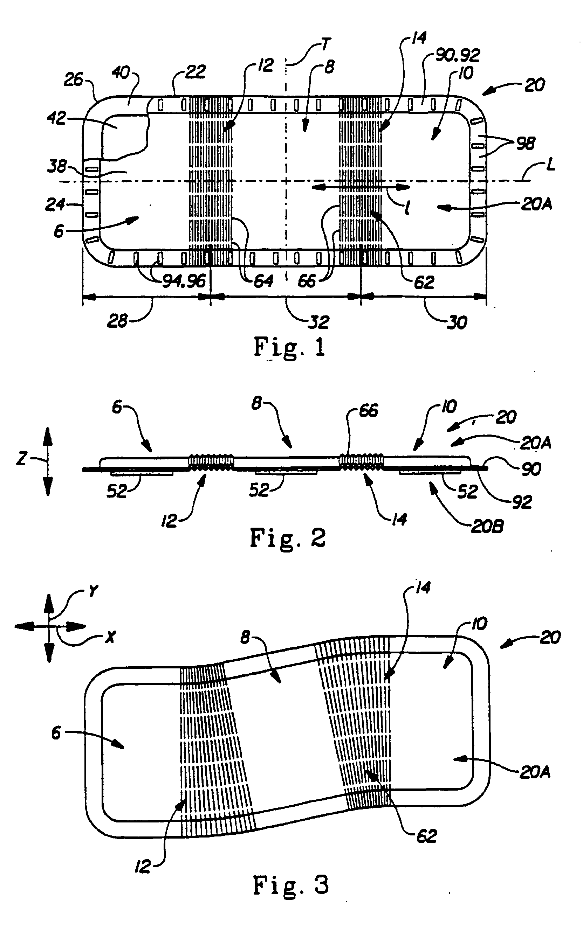

The present invention relates to absorbent articles, such as sanitary napkins, panty liners, and incontinence pads. FIGS. 1-4 are simplified illustrations of one preferred embodiment of a disposable absorbent article of the present invention, sanitary napkin 20. The sanitary napkin 20 has a first portion 6, a second portion 8, and a third portion 10. The first portion 6 and second portion 8 are separated by an expandable portion, in the form of a flexible and extensible expansion joint, which will be referred to as first intermediate portion 12. The second portion 8 and third portion 10 are similarly separated by an expandable portion, flexible and extensible second intermediate portion 14.

The sanitary napkin 20 has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 (shown in FIG. 2) is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows that the sanitary napkin 20 has two spaced apart longitudinal edges 22, two spaced apart transverse or end edges (or 'ends') 24, which together form the periphery 26 of the sanitary napkin. The sanitary napkin also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the sanitary napkin 20. A more detailed description of the characteristics of a central region and two end regions for a sanitary napkin is contained in U.S. Patent 4,690,680 issued to Higgins on September 1, 1987.

The sanitary napkin 20 can be of any thickness, including relatively thick, relatively thin, or even very thin. The embodiment of the sanitary napkin 20 shown in Figures 1-4 of the drawings is intended to be an example of a relatively thin sanitary napkin, preferably an "ultra-thin" sanitary napkin as described in U.S. Patents 4,950,264 and 5,009,653 issued to Osborn. The sanitary napkin 20 shown should preferably also be relatively flexible, so that it is comfortable for the wearer. It should be understood that the sanitary napkin shown in FIGS. 1-4 is merely one preferred embodiment of the present invention, and that the present invention is not limited to absorbent articles of the type or having the specific configurations shown in the drawings.

FIG. 1 shows the individual components of the sanitary napkin 20 of the present invention. The sanitary napkin 20 shown in FIG. 1 generally comprises at least three primary components. These include a liquid pervious topsheet 38, a liquid impervious backsheet 40, and an absorbent core 42 positioned between the topsheet 38 and the backsheet 40. The topsheet, backsheet, and absorbent core can comprise many of the basic materials commonly used for such purposes. Such materials can either be relatively inextensible, or relatively extensible under the forces associated with wearing the sanitary napkin. Preferably, however, if such materials are relatively inextensible, at least portions of such materials are mechanically modified or otherwise altered as described in greater detail below to provide the sanitary napkin with the flexible and extensible intermediate portions 12 and 14.

In the preferred embodiment shown in FIGS. 1-4, the topsheet 38 comprises an apertured formed film made in accordance with U.S. Patent 4,342,314 issued to Radel, et al. and U.S. Patent 4,463,045 issued to Ahr, et al. The absorbent core 42 comprises a laminate of absorbent gelling material (or "superabsorbent hydrogelforming material") placed between two layers of air-laid tissue. The backsheet 40 comprises a polyethylene film. The sanitary napkin 20 shown in FIGS. 1-4 is assembled in a sandwich construction in which the topsheet 38 and the backsheet 40 have length and width dimensions generally larger than those of the absorbent core 42. The topsheet 38 and the backsheet 40 extend beyond the edges of the absorbent core 42 and are joined together to form portions of the periphery 26 of the sanitary napkin 20.

The first portion 6, second portion 8, and third portion 10, comprise the relatively inextensible portions of the sanitary napkin. Because of their location in the sanitary napkin, the first portion 6, second portion 8, and thi rd port ion 10 may also be referred to as a front portion, central portion, and rear portion, respectively. These portions can comprise parts of the sanitary napkin that are made of conventional materials which are assembled in a conventional manner and are otherwise unaltered by mechanical modifications. These relatively inextensible portions of the sanitary napkin may, thus, have the flexibility and very low extensibility of any currently marketed sanitary napkins. Suitable flexibilities for some of the more flexible currently marketed sanitary napkins are described in U.S. Patents 4,950,264 and 5,009,653 issued to Osborn. Currently marketed sanitary napkins typically are relatively inextensible under the forces acting on the sanitary napkin during wear. Typically, such sanitary napkins are less than about 5% extensible under stretching forces of between about 50 grams_{f} to about 1,500 grams_{f} without destroying the integrity of the sanitary napkin.

In alternative embodiments, however, instead of being relatively inextensible, the first portion 6, second portion 8, and third portion 10, can be provided with greater extensibility than conventional sanitary napkins (e.g., greater than or equal to about 5% extensibility under the above range of forces). If the first, second, and third portions 6, 8, and 10 are provided with a degree of extensibility, these portions of the sanitary napkin 20 should still be less flexible and extensible than the flexible and extensible intermediate portions 12 and 14. Because of this, the first, second, and third portions 6, 8, and 10, of the sanitary napkin 20 may be referred to as the "less extensible portions" of the sanitary napkin. The term "less extensible portions" will, therefore, cover situations where these first, second, and third portions are relatively inextensible, and situations where they are extensible, but less extensible that the extensible intermediate portions.

The flexible and extensible intermediate portions 12 and 14 may be any suitable type of structure of the sanitary napkin that is more flexible and extensible than the adjacent less extensible portions of the sanitary napkin. The intermediate portions 12 and 14 provide the sanitary napkin with regions of extensibility (and preferably, stretchability) so that the sanitary napkin 20 can stretch with the wearer's panties and be more comfortable when placed adjacent to the wearer's body. The intermediate portions 12 and 14 also provide the advantage that they serve as flexible and extensible expansion joints about which the adjacent less extensible portions can articulate to conform to the different regions of the wearer's body.

The extensibility of the intermediate portions 12 and 14 of the sanitary napkin 20 is shown in FIG. 3. The terms "extensible" or "expandable", as used herein refer to articles or at least portions of entire absorbent articles, that can be increased in at least one of their dimensions in the X-Y plane. The X-Y plane, as defined herein, is a plane that is oriented generally parallel to the faces 20A and 20B of the sanitary napkin 20. The X axis, as shown in FIG. 3, is longitudinally oriented (parallel to the longitudinal centerline, L) and the Y axis is transversely oriented (parallel to the transverse centerline, T). Since the extensibility referred to herein is in the X-Y plane, it can be distinguished from any increase in length of a curved absorbent article that results from straightening such an article in order to place it into a flat configuration. The extensibility referred to herein, since it is generally planar, can also be distinguished from any increase in length the may result from the mere unfolding of a folded absorbent article or components thereof.

The expandable portions, such as intermediate portions 12 and 14, may contain, but are preferably free of folded overlapping sections that are stacked on top of one another in the Z-direction. Any ridges formed in the expandable portions 12 and 14 are preferably arranged in a generally side-by-side relationship, and are preferably so small in height that they do not appreciably alter the surface characteristics of the sanitary napkin or deviate from the plane of the topsheet. Any such ridges are preferably less than or equal to about 1.5 mm in height, and more preferably less than or equal to about 1 mm in height. This will enable the expandable portions to extend and contract in use without any gross movements, such as unfolding, that would be noticed by and objectionable to the wearer.

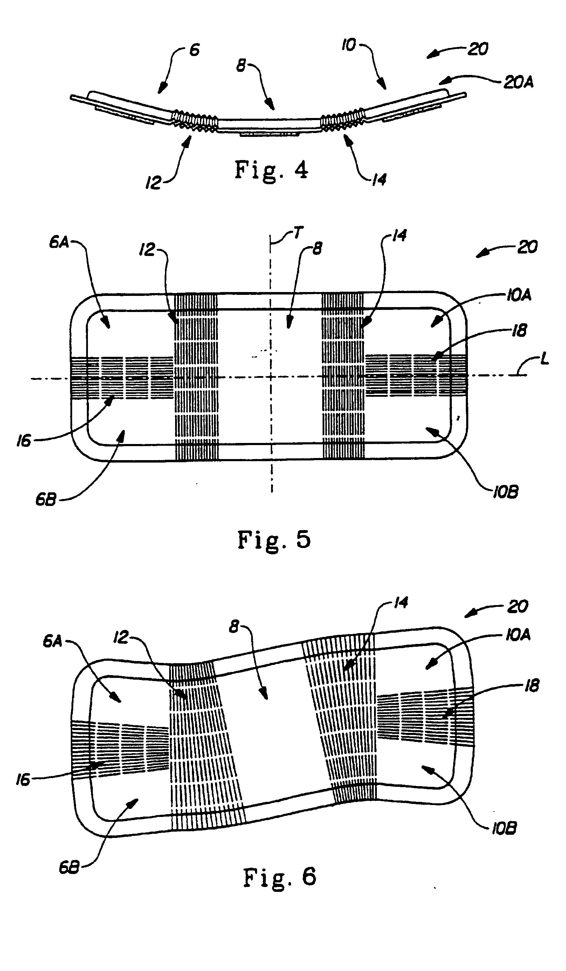

The flexibility of the intermediate portions 12 and 14 also allows the less extensible first, second, and third portions, 6, 8, and 10, to articulate in the Z-direction as shown in FIG. 4. The presence of the intermediate portions 12 and 14 allows the less extensible portions to articulate in any manner described in the aforementioned PCT Publication WO 92/10984 (allowed U.S. Patent Application Serial No. 08/129,900), now U.S. Patent 5,484,430 entitled "Sanitary Napkin Having Transversely Segmented Core". The less extensible portions can also assume any of the configurations disclosed in the aforementioned EPO Publication No. 0 605 017 A2 published in the name of Tong-ho J. Hsieh.

The intermediate portions 12 and 14, however, may, in addition, provide the sanitary napkin 20 with an overall extensibility, which is not present, in the sanitary napkin shown in the above described EPO reference published in the name of Tong-ho J. Hsieh. More specifically, the sanitary napkin shown in EPO Publication 0 605 017 A2 is described as a structure which allows the front and rear portions of the article to freely bend upward into contact with the user's body. The structure of the sanitary napkin is said to also prevent lateral compression in the central portion of the sanitary napkin from being transmitted to the front and rear portions of the sanitary napkin. The front and rear portions of conventional sanitary napkins are said to undesirably increase the stiffness of the article in the longitudinal direction which makes it more difficult for such sanitary napkins to assume the desired curved configuration.

The EPO publication describes the article as having an "expansion joint" formed between first and second portions of the sanitary napkin. However, a careful review of the EPO publication reveals that no true "expansion" in the form of elongation of the sanitary napkin occurs at these joints. The "expansion joints" described in the EPO publication comprise portions of the sanitary napkin where notches are provided in the absorbent core to create preferential bending axes so that the sanitary napkin can assume a curved configuration. The fluid pervious cover is provided with a slit in the portions of the cover that lies within each of the notches. The fluid impervious barrier layer has a pleat formed therein which is able to expand in the portion of the barrier that lies between the edges of the notches. Thus, the only portion of the sanitary napkin shown in the EPO reference that could be considered to "expand" is the pleated portion of the fluid impervious barrier that lies with the notches on the sides of the product. That portion of the barrier will only "expand" when the front and rear portions of the sanitary napkin fold upward to place the sanitary napkin in a curved configuration, or when the central portion of the sanitary napkin is compressed and the pleated section of the barrier rotates to isolate this compression from the front and rear portions of the sanitary napkin. There is no overall extensibility of the product in the longitudinal direction along the longitudinal centerline of the product due to the "expansion joints" because the absorbent core is generally inextensible along the longitudinal centerline of the product.

The extensible intermediate portions 12 and 14 of the sanitary napkin 20 of the present invention shown in FIGS. 1-4, however, unlike the product described in the EPO publication, provides the sanitary napkin with regions that are extensible across the full width of the sanitary napkin. The extensible intermediate portions 12 and 14 of the sanitary napkin are preferably each extensible in an amount that is greater than about 5% up to about 50%, more preferably between about 10% and about 40%, and most preferably between about 25% and about 40% under the forces associated with wearing the sanitary napkin in a pair of panties. Therefore, the sanitary napkin is preferably capable of such extension under forces of between about 50 - 100 grams to about 1,000 - 1,500 grams, more preferably under forces of between about 250 grams and about 800 grams. In this regard, it should be understood that all of the limits and ranges specified herein include all narrower ranges, limits, and amounts tht are within the specified limits and ranges. The extensible intermediate portions 12 and 14 also provide the sanitary napkin with an overall extensibility.

The sanitary napkin 20, as shown in FIG. 3, is preferably extensible at least in the longitudinal direction. FIG. 3 also shows that different portions of the extensible regions 12 and 14 can extend in different amounts so that the less extensible portions of the sanitary napkin can shift laterally relative to each other. For example, the first portion 6 is capable of moving so that it may be displaced laterally relative to the second portion 8 so that the longitudinal side edge of one side of the first portion is shifted laterally outboard of the longitudinal side edge of the same side of the second portion 8. This is useful in providing the sanitary napkin with the ability to more closely conform to the wearer's body and the wearer's panties when the wearer's body goes through a range of body movements.

In other embodiments, the sanitary napkin 20 may have extensible portions that are extensible in the transverse (or "y-direction"), or in more than one direction. For example, the sanitary napkin 20 shown in Figures 5 and 6 has both regions that are extensible in the longitudinal direction (extensible portions 12 and 14), and in the transverse direction (transversely extensible intermediate portions 16 and 18). (In this regard, it should be understood that although the description may primarily refer to the extensible intermediate portions 12 and 14 of the embodiment shown in FIGS. 1-4 for simplicity, the characteristics decribed herein of the extensible intermediate portions may also be applicable to any of the other expandable portions shown and described herein.)

The extensible portions 12 and 14 of the sanitary napkin 20 may in some preferred embodiments, in addition to being extensible, also be stretchable. The term "stretchable", as used herein, refers to articles, or portions thereof, that are extensible when stretching forces are applied to the article and offer some resistance to stretching. More preferably still, the extensible portions 12 and 14 of the sanitary napkin 20 may be elastically stretchable. The terms "elastically stretchable' or "elastically extensible' are intended to be synonymous. These terms, as used herein, mean that when the stretching forces are removed, these portions of the sanitary napkin will tend to return toward their unextended or unstretched (or "original" dimensions). The extensible portions 12 and 14 of the sanitary napkin 20 need not return all the way to their unstretched dimensions, however. These extensible portions may return to relaxed dimensions between their unstretched dimensions and extended (or stretched dimensions). Preferably, the extensible portions 12 and 14 are made elastically stretchable without utilizing conventional elastics in which elastic strands are extended and attached to generally inextensible components. Making the extensible portions 12 and 14 of the sanitary napkin elastically stretchable will reduce the undesirable tendency of the lengthened extensible portions 12 and 14 of the sanitary napkin to develop excessive slack therein when the forces which tend to stretch the sanitary napkin are removed. This is particularly of interest when the wearer's panties contract after extension.

The extensible portion 12 and 14 of the embodiment shown in FIGS 1-4 are located in regions adjacent to, and containing, the boundary between each of the end regions, 28 and 30, and the central region 32. This allows the less extensible first, second, and third regions, 6, 8, and 10, to move and articulate relative to each other to conform to the distinct regions of the wearer's body that are described in greater detail in the aforementioned patent application entitled "Sanitary Napkin Having Transversely Segmented Core".

The size of the expandable portions useful in the various embodiments described herein can vary widely. According to the present invention, as shown in the embodiment shown in FIGS. 1-4, the dimension of the expandable portions measured in the transverse or lateral direction corresponds to that of the full width of the sanitary napkin. The dimension of the expandable portions measured in the longitudinal direction can, likewise, extend only a portion of the length of the sanitary napkin. According to the present invention, as shown in the embodiment shown in FIGS. 1-4, the dimension of the extensible intermediate portions measured in the longitudinal direction is less than the full length of the sanitary napkin, and preferably, is substantially less than the full length of the sanitary napkin so that the sanitary napkin will be easier to manufacture. Suitable dimensions measured in the longitudinal direction for the extensible intermediate portions, for example, may be less than or equal to about 1 inch (about 2.5 cm).

As noted above, the extensible portions 12 and 14 in the embodiment shown in FIGS. 1-4 may, and preferably do, provide the sanitary napkin 20 with an overall extensibility. The sanitary napkin 20 is preferably extensible in the amounts described in P&G PCT Publication No. WO 93/01785. To summarize the same, the sanitary napkin is preferably capable of extending about 5% to less than about 50%. The sanitary napkin of the present invention can also be provided with any of the other features of the sanitary napkins described in the PCT Publication No. WO 93/01785 including, a structure that provides a "force wall" to prevent elongation past a certain amount without substantial increases in the amount of force applied to the sanitary napkin.

The flexible and extensible portions 12 and 14 can comprise any suitable type of structure that provides the sanitary napkin with the properties described herein. The flexible and extensible portions 12 and 14 can be integral with one or more of the components of the sanitary napkin, or separate elements that are joined thereto. For example, suitable structures for the flexible and extensible intermediate portions 12 and 14 could comprise one or more separate pieces of material that are more flexible and extensible than the material comprising the adjacent portions. Such separate elements or pieces could be joined to and bridge the adjacent first, second, and third portions of the sanitary napkin. Such a material can be a material that is inherently more flexible and extensible than the material comprising the adjacent portions (e.g., an elastic material), or it can be some suitable material that is folded, pleated, or otherwise mechanically modified to provide it with greater flexibility and extensibility.

Alternatively, the extensible portions 12 and 14 can be integral with the various layers or components of the adjacent portions, but which are physically (e.g., mechanically, chemically, or otherwise) modified to provide them with greater flexibility and extensibility than the portions of such a material that comprise the less extensible adjacent portions. Preferably, the extensible portions 12 and 14 are formed in this manner for ease of manufacture. The extensible portions 12 and 14 can be mechanically modified at any suitable stage of the manufacturing process. The extensible portions 12 and 14 can be provided with flexibility and extensibility by either modifying one or more of the individual components of the sanitary napkin before assembling such components together to form the sanitary napkin, or by mechanically modifying regions of the sanitary napkin after the sanitary napkin has been completely assembled. Preferably, the topsheet 38 and backsheet 40 are mechanically modified in separate operations prior to assembling the sanitary napkin 20.

The portions of the topsheet 38 and backsheet 40 that comprise part of the extensible portions 12 and 14 may be provided with increased flexibility and extensibility by creping, or alternatively, by ring rolling (or pre-corrugating) desired regions of the topsheet 38 and backsheet 40. Suitable methods for ring rolling are described in U.S. Patent 4,107,364 issued to Sisson on August 15, 1978, U.S. Patent 4,834,741 issued to Sabee on May 30, 1989, U.S. Patent 5,143,679 issued to Gerald M. Weber, et al. on September 1, 1992, U.S. Patent 5,156,793 issued to Kenneth B. Buell, et al. on October 20, 1992, and U.S. Patent 5,167,897 issued to Gerald M. Weber, et al. on December 1, 1992.

More preferably, the portions of the topsheet and backsheet 40 that comprise part of the extensible portions 12 and 14 are provided with increased flexibility and extensibility by forming strainable networks in the desired regions of the topsheet 38 and backsheet 40. FIGS. 1-4 show the strainable network 62 that is formed in the portions of the topsheet 38 and backsheet 40 that lie in the extensible portions 12 and 14 of the sanitary napkin 20. The process of forming a strainable network in a material is particularly preferred for this purpose because (like ring rolling) such an operation can be readily adapted for use in high speed manufacturing operations. There is no need to pleat or fold the entire absorbent article, or portions thereof. Further, the process of forming a strainable network in a material is highly preferred because it can be adapted to produce a virtually unlimited number of patterns to tailor the extensibility of the strainable network. This process can also provide the extensible regions of the sanitary napkin with elastic-like stretchability without the use of added elastic materials. Because the base material into which the strainable network is formed is often a film (or has at least one component which is a film), the formed material may be referred to herein for convenience as a Structural Elastic-Like Film or "SELF" material.

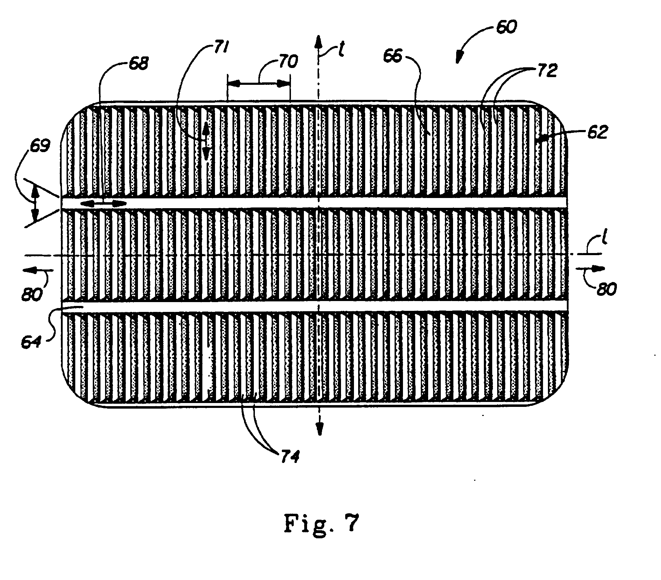

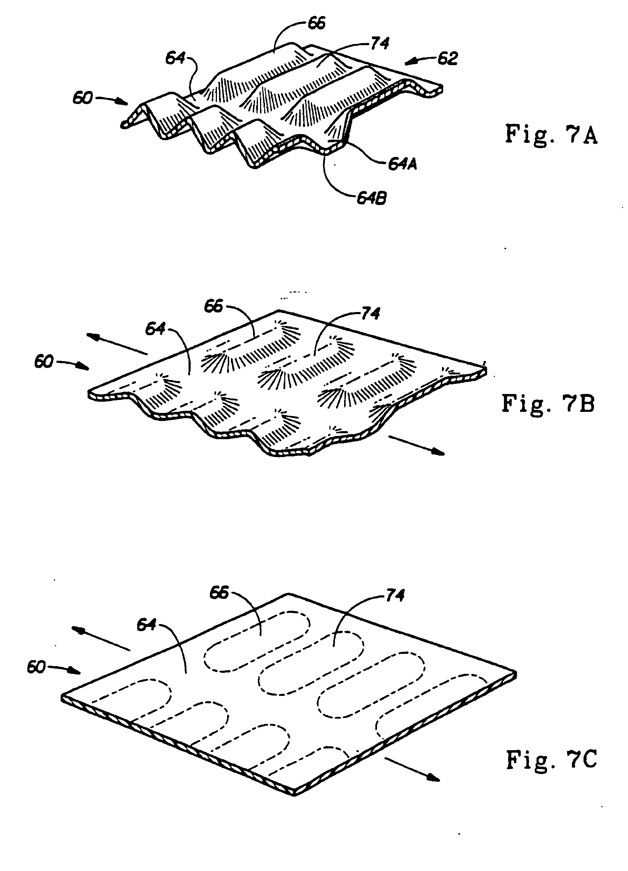

The characteristics of the strainable network 62 of such a SELF material will be discussed with reference to FIGS. 7-7C. FIGS. 7-7C show enlarged views of a web material 60 having a strainable network 62 formed therein. The web material 60 shown in FIGS. 7-7C can comprise a portion of the topsheet 38 or backsheet 40 in the intermediate portions 12 and 14 of the sanitary napkin. (Alternatively, the entire topsheet and/or backsheet of the sanitary napkin can comprise a "SELF" web material 60 having a strainable network formed therein.) To the extent the web material 60 shown in FIGS. 7-7C comprises a topsheet material, the web material is shown in a form without any apertures for simplicity. FIGS. 7 and 7A show the web material 60 in an untensioned condition. The web material 60 having a strainable network 62 formed therein has a longitudinal centerline (or axis), 1, and a lateral centerline (or axis), t. The longitudinal centerline, 1, is shown in FIG. 7 as being rectilinear. However, the longitudinal centerline, 1, is not limited to such a configuration. The longitudinal centerline, 1, can be rectilinear, curvilinear, or partially rectilinear and partially curvilinear.

FIGS. 7 and 7A show that the web material 60 which includes the strainable network 62 comprises at least two distinct and dissimilar regions. The term "strainable network', as used herein, refers to an interconnected and interrelated group of regions which are able to be extended to some useful degree in a predetermined direction. FIGS. 7 and 7A show that the two distinct regions of the strainable network 62 comprise at least a first region 64 and a second region 66. The two distinct regions provide the web material 60 with a first elastic-like, relatively low resistive force stage to stretching forces, and a second relatively high resistive force stage. The strainable network 62 is created by forming the network into the web material 60. As used herein, the term "formed" refers to the creation of a desired structure or geometry upon the web material 60 that will substantially retain the desired structure or geometry when it is not subjected to any externally applied elongations or forces. Suitable methods for forming a strainable network into a web material include, but are not limited to embossing by mating plates or rolls, thermoforming, high pressure hydraulic forming, or casting.

The strainable network 62 is configured so that the first region 64 will exhibit resistive forces in response to an applied axial elongation in a direction parallel to a predetermined axis (which, in this case, is the longitudinal axis l) before a substantial portion of the second region 66 develops significant resistive forces to the applied elongation. The first and second regions, 64 and 66, of the strainable network 62 each have a first surface and an opposing surface. The configuration of the web material 60 having a strainable network 62 formed therein is described in terms the surface-pathlengths (i. e., distance measured across topography) of the different regions. As shown in FIG. 7A, the first region 64 has a surface-pathlength which is less than that of the second region 66. The surface-pathlengths are measured substantially parallel to the predetermined axis over the topography of the surface of each region while the material is in an untensioned condition. As shown in FIG. 7A, the second region 66 includes one or more deformations 74 which extend beyond the plane of the first region 64.

In the preferred embodiment shown in FIGS. 7 and 7A, the strainable network 62 includes a plurality of first regions 64 and a plurality of second regions 66. In the preferred embodiment shown in FIGS. 7 and 7A, the first regions 64 are substantially planar regions. That is, the material within the first region 64 is in substantially the same condition before and after the formation step undergone by web material 60. The second regions 66 include a plurality of continuous, interconnected, deformations 74 which extend alternately beyond the plane of both the first and second surfaces (64A and 64B, respectively) of the first region 64. In other embodiments, the deformations 74 may extend beyond the plane of only one of the first 64A or the second 64B surfaces of the first region 64.

FIG. 7 shows that the first regions 64 of the strainable network 62 have a first axis 68 and a second axis 69, wherein the first axis 68 is preferably longer than the second axis 69. In the simplified embodiment shown, the first axis 68 of the first region 64 is substantially parallel to the longitudinal axis, 1, of the strainable web material 60 while the second axis 69 is substantially parallel to the transverse axis, t, of the strainable web material 60. The second regions 66 of the strainable network 62 also have a first axis 70 and a second axis 71. The first axis 70 of the second region 66 is substantially parallel to the longitudinal axis 1 of the web material 60, while the second axis 71 is substantially parallel to the transverse axis t of the web material 60. In the version of the web material shown in FIGS. 7 and 7A, the first regions 64 and the second regions 66 are substantially linear, extending continuously in a direction substantially parallel to the longitudinal axis 1 of the strainable web material. In the sanitary napkin embodiment shown in FIGS. 1-4, the longitudinal centerline 1 of the strainable network 62 in the web material, topsheet 38 and backsheet 40, is generally oriented in the longitudinal direction. In other embodiments, however, the longitudinal centerline 1 of the web material can be oriented in other directions, depending on the direction of extensibility desired.

FIGS. 7A, B, and C show the manner in which the web material 60 with the strainable network 62 exhibits at least two significantly different stages of controlled resistive force to elongation when subjected to an applied elongation in a direction parallel to a predetermined axis. The strainable network 62 exhibits first resistive forces to the applied elongation (which develop between the stage shown in FIG. 7A and the stage shown in FIG. 7B). The first resistive forces occur until the elongation of the web is sufficient to cause a substantial portion of the second regions 66 to enter the plane of applied elongation, as shown in FIG. 7B. After the web material 60 reaches the stage shown in FIG. 7B, it exhibits second resistive forces to further elongation (as illustrated by FIG. 7C). Typically, when used in the topsheet 38 and backsheet 40 of the sanitary napkin of the present invention, the web material will be within the first stage of resistance to elongation so the various portions of the strainable network 62 will only extend to the stage shown in FIG. 7B and adjust so as to relax back to the stage shown in FIG. 7A.

The topsheet and backsheet material into which the strainable network 62 is formed can comprise a base material that has a relatively low extensibility under the forces that the sanitary napkin is normally subjected to when worn. When the strainable network 62 is formed therein, however, the base material will be extensible under the forces the sanitary napkin is normally subjected to when worn. The base material used as the topsheet 38 and backsheet 40 can respectively comprise the apertured formed film and the polyethylene film, described above. These base materials, however, are preferably comprised substantially of linear low density polyethylene (LLDPE). The base materials may also be comprised of other polyolefins such as polyethylenes, including low density polyethylene (LDPE), ultra low density polyethylene (ULDPE), high density polyethylene (HDPE), or polypropylene and blends thereof with the above and other materials. Examples of other suitable polymeric materials which may also be used as base materials include, but are not limited to polyester, polyurethanes, compostable or biodegradable polymers, heat shrink polymers, thermoplastic elastomers, and breathable polymeric structures.

The depth and number of deformations 74 in the strainable network 62 can be varied to control the applied force or elongation required to extend the extensible portions of the topsheet 38 and backsheet 40. In one preferred embodiment, the deformations 74 are formed by two rigid plates having outer dimension of 5.0" by 12" by 0.75" (12.7 cm by 30.5 cm by 2 cm). On one surface of each plate are a series of meshing teeth which are substantially triangular in cross section and measure 0.030" (0.76 mm) at their bases and taper to a vertex with a radius of 0.008" (0.2 mm) at the top. The centerlines of the teeth are spaced evenly and at 0.030" (0.76 mm) increments. On the "toothed" side of one plate, a series of grooves are cut which are parallel to each other and perpendicular to the evenly spaced teeth. These grooves measure 0.031" (0.8 mm) wide and are continuous over the entire length of the plate, and are spaced at a distance of 0.25" (6.4 mm) on center. These grooves correspond to the undeformed regions of the base material. The preferred base material is placed between the plates in a hydraulic press having platens larger than the plates to evenly distribute pressure. The plates are compressed under a load of at least 4,000 pounds (1,800 Kg). The formed web material is then removed from between the plates. The available stretch or elongation is increased if for a given number of deformations, the height or degree of deformation imparted to the web material is increased. Similarly, the available stretch or elongation is increased if for a given height or degree of deformation, the number or frequency of deformations is increased. Methods for forming a strainable network into a portion of a material such as a topsheet or backsheet suitable for the sanitary napkin 20 of the present invention are described in greater detail in U. S. Patent Application Serial Number 08/203,087 entitled "Web Materials Exhibiting Elastic-Like Behavior" filed in the name of Chappell, et al. (PCT Publication No. WO 95/03765 published February 9, 1995).

The above-described method for providing the topsheet 38 and backsheet 40 with a strainable network is particularly well suited to providing the sanitary napkin 20 with different regions of extensibility. The mating plates can be configured to create any of the patterns of extensibility within the topsheet 38 and backsheet 40 of the sanitary napkins 20 shown in the drawings. If desired, it is also possible to eliminate the first regions 64 of the strainable network from the topsheet and backsheet (in which case the second regions 66 will be continuous). The elimination of the first regions 64 has the effect of substantially reducing or eliminating the elastic-like tendency for the various regions of the topsheet and backsheet to retract (and, thus, to also reduce or eliminate the two different stages of controlled resistive force to elongation). Having looked at the characteristics that allow regions of the tops and backsheet to be extensible in the intermediate regions 12 and 14 of the sanitary napkin 20, the characteristics of the absorbent core and the assembly of the same in the sanitary napkin will now be examined.

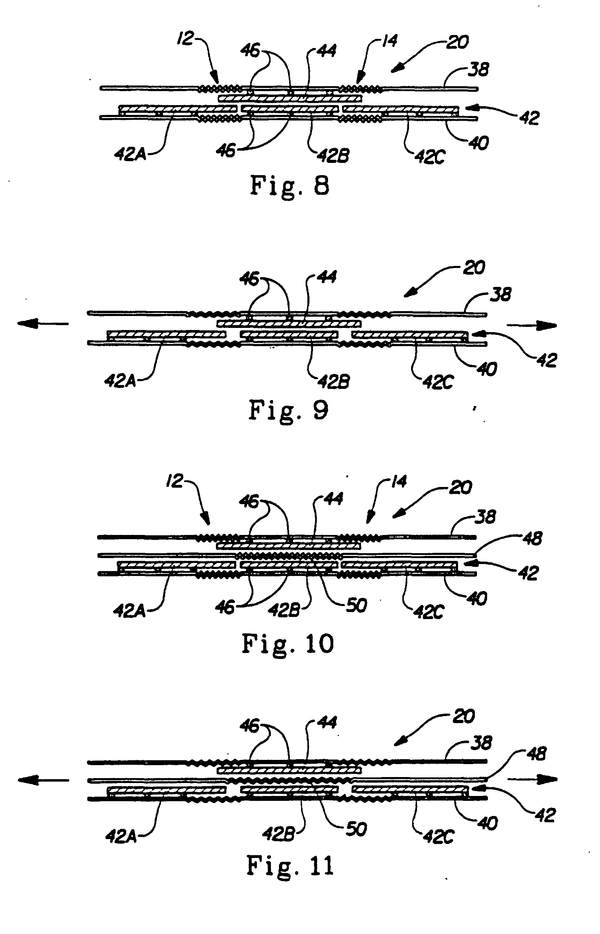

FIGS. 8 and 9 show one preferred way of providing an absorbent core 42 for the embodiment shown in FIGS. 1-4 that will be extensible in the intermediate portions 12 and 14 of the sanitary napkin 20. In the embodiment shown in FIGS. 8 and 9, the absorbent core 42 is transversely divided into a plurality of independent segments, 42A, 42B, and 42C. The sanitary napkin 20 is also preferably provided with one or more overlying absorbent layers 44 that span the gaps between the adjacent core segments 42A, 42B, and 42C. The overlying layer or layers 44 may be considered to comprise part of the absorbent core 42, or as a separate component of the sanitary napkin.

FIGS. 8 and 9 show that the overlying layer 44 is preferably joined to the topsheet 38 between the SELFed regions of the topsheet so that the inextensibility, or lesser extensibility of the overlying layer 44 does not interfere with the extensibility of the SELFed intermediate regions 12 and 14. The joinder can be accomplished in any suitable manner, such as by adhesive bonds 46. The underlying independent core segments 42A, 42B, and 42C are similarly joined to the backsheet 40 between the SELFed regions of the same. The overlying absorbent layer 44, however, is preferably not joined to the independent core segments 42A, 42B, and 42C or to any other component of the sanitary napkin 20 inside the periphery of the sanitary napkin 20 so that the overlying absorbent component 44 can slide freely relative to the independent absorbent core segments 42A, 42B, and 42C when the sanitary napkin is stretched as shown in FIG. 9. The term "joined", as used herein, encompasses configurations in which an element is directly secured to another element by affixing the element directly to the other element; configurations in which the element is indirectly secured to the other element by affixing the element to intermediate members) which in turn are affixed to the other element; and configurations in which one element is integral with another element, i.e., one element is essentially part of the other element.

The absorbent components of the sanitary napkin 20 such as the independent core segments and the overlying absorbent layer can be joined to the desired component of the sanitary napkin, such as the topsheet and backsheet in any suitable manner. Suitable means include joining such components by adhesives, stitching, heat and/or pressure bonds, dynamic mechanical bonds, ultrasonic bonds, intermingling or entanglement of the fibers or other structural elements comprising the components of the sanitary napkin, such as by meltblowing the fibers comprising one component onto another component, extruding one component onto another, or by any other means known in the art. Several of these means for attaching the components of the sanitary napkin are described in greater detail in U.S. Patent Application Serial No. 07/810,744 filed in the name of Cree, et al. on December 17, 1991 (PCT Patent Publication No. WO 93/11725 published on June 24, 1993).

The topsheet 38 and backsheet 40 can be joined together in any suitable manner that allows the intermediate portions 12 and 14 of the sanitary napkin to extend. As shown in Figure 1, the topsheet 38 is preferably secured to backsheet 40 along a peripheral edge seal, such as seal 90. Seal 90 is preferably liquid impervious. The seal 90 can be formed by any means commonly used in the art for this purpose such as by gluing, crimping, or heat-sealing. There are a number of approaches that can be used to produce fluid impermeable, stretchable edge seals between the topsheet 38 and the backsheet 40.

In a particularly preferred sanitary napkin embodiment shown in FIGS. 1-4, the portions of the topsheet 38 and backsheet 40 along the edges of the topsheet and backsheet 40 are joined together using an extensible adhesive 92 around the perimeter of the sanitary napkin and in addition, a preferred distribution of mechanical bonds 94 in the perimeter area. The extensible adhesive 92 provides an extensible impervious seal around the perimeter 26 of the sanitary napkin. The mechanical bonds 94 provide added strength. The mechanical bonds 94 are arranged in intermittent zones (or regions) of bonded areas 96 and bonded areas 98. In the embodiment shown in FIG. 1, the bonded areas 96 comprise a plurality of spaced apart line segments that are approximately perpendicular to the periphery 26 of the sanitary napkin 20. The bonded areas 96 should be spaced apart in the desired direction of extensibility for the completely assembled absorbent article and be separated by unbonded areas 98. The bonded areas 96 are not extensible and the unbonded areas 98 are extensible. The bonded areas 96 are preferably formed by a heat and pressure process in which the application of temperature and pressure are controlled so that neither the material comprising the topsheet nor the material comprising the backsheet are melted in the process. A suitable seal of a polyethylene formed film topsheet having SELFed regions therein and a polyethylene film backsheet having SELFed regions therein can be formed by a heated plate having raised areas in the pattern of the bonded areas shown in Fig. 1 which are approximately 6 mm x 2 mm which are spaced 5 mm apart. The plate is heated to 170°F for 5 seconds and the seal is formed using hand pressure. Other methods suitable for joining the components of an extensible absorbent article are described in greater detail in U.S. Patent Application Serial No. 08/192,240 filed in the name of Osborn, et al. (PCT Publication No. WO 95/20931).

The above manners of joining the components of the sanitary napkin 20 are preferred for ease of construction. (Other means of uniting the various components can be used.) For instance, the present invention also includes so-called 'tube' products. In these products, a liquid pervious cover material (such as topsheet material) can be wrapped completely around the absorbent core and the backsheet, and then the components can be secured together. In alternative arrangements, the topsheet could be wrapped around the core, and the wrapped core could be placed on and secured to the backsheet.

FIGS. 10 and 11 show a highly preferred additional feature that for the sanitary napkin 20 shown in the preceding figures. As shown in FIGS. 10 and 11, the sanitary napkin 20 can additionally be provided with a component such as a separation component (or "stabilization layer") 48 that is positioned between the overlying absorbent layer 44 and the independent absorbent core segments 42A, 42B, and 42C. The stabilization layer 48 serves to prevent any undesirable tendency for interference to occur between the ends of the independent core segments and the overlying absorbent component 44 when these components slide past each other. That is, the stabilization layer 48 prevents the ends of the independent core segments 42A, 42B, and 42C and the overlying absorbent component 44 from engaging each other so as to prevent such a sliding motion.

Preferably, as shown in FIGS. 10 and 11, the stabilization layer 48 is provided in a form that facilitates the relative sliding action between the independent core segments 42A, 42B, and 42C and the overlying absorbent component 44. The stabilization layer 48 is preferably liquid pervious so that body exudates can pass through the stabilization layer 48 to the underlying absorbent core segments 42A, 42B, and 42C. The stabilization layer 48 can be made of any material that satisfies these criteria. Suitable materials for use as the stabilization layer 48 include, but are not limited to nonwoven webs at least a portion of which are creped, extensible scrims; or extensible apertured films. Two particularly preferred nonwoven webs are an air-through bonded nonwoven material comprised of bi-component fibers which is manufactured under the tradename HAVIX S2156 by the Havix Corporation, Gifu-City Japan (formerly known as Fukumura) which is micrex creped which can be performed by the Micrex Corporation of Walpole, MA, and a nonwoven web known as CORO-LIND PE, which was obtained from Corovin GMBH of Germany and then SELFed.

The stabilizing layer 48 is preferably joined at its ends to one or more of the adjacent components of the sanitary napkin and is preferably not secured to any other component in the central region 32 of the sanitary napkin. As shown in FIGS. 10 and 11, the stabilizing layer 48 can be provided with extensibility, such as by creping or SELFing a region of the same. The extensible region 50 of the stabilizing layer 48 can be located on any suitable portion of the stabilizing layer. Preferably, however, the extensible region 50 is located in a central region of the stabilizing layer 48 so that when the sanitary napkin stretches as shown in FIG. 10, the extensible region 50 bridges the gaps between the adjacent independent core segments 42A, 42B, and 42C. Having, thus, described the assembly of the components of a preferred sanitary napkin embodiment, some of the additional features that are preferably provided on the sanitary napkin 20 will be examined.

The garment surface 20B of the sanitary napkin 20 may include, and preferably does include, fasteners for attaching the sanitary napkin to the wearer's undergarments. Figure 2 shows the fastener 52 which is adapted to secure the sanitary napkin to the crotch region of an undergarment. Fasteners comprising adhesives have been found to work well for this purpose, with pressure sensitive adhesives being preferred. The adhesive fastener is typically covered with a removable cover strip or release liner in order to keep the adhesive from sticking to a surface other than the crotch portion of the panty prior to use. Suitable adhesives and release liners are described in greater detail in U.S. Patent 4,917,697. One preferred release liner which also serves as a package for individually wrapping a sanitary napkin is described in U.S. Patent 4,556,146 issued to Swanson. The sanitary napkin 20 of the present invention is used by removing any release liner and placing the sanitary napkin 20 in a panty so that the adhesive (or other fastener) 52 contacts the panty and maintains the sanitary napkin in position within the panty during use.

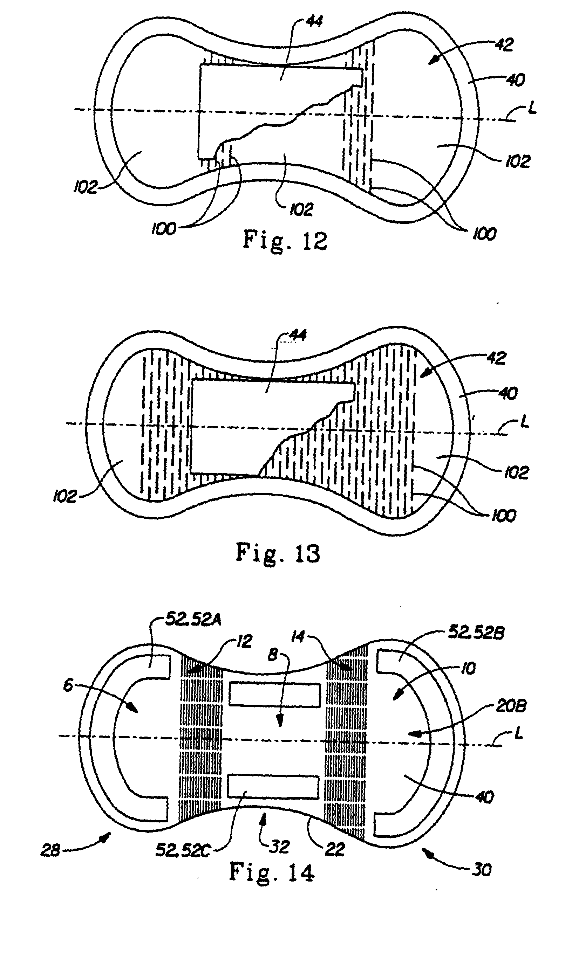

Various alternative embodiments of the sanitary napkin of the present invention are also possible. FIG. 12, for example, shows a backsheet 40 and absorbent core 42 that are in a particularly preferred shape for the sanitary napkin of the present invention. This preferred shape, and the advantages thereof, are described in greater detail in the aforementioned U.S. Patent Application Serial No. 08/192,240 (PCT Publication No. WO 95/20931). FIG. 12 also shows an alternative absorbent core 42 embodiment in which the absorbent core is provided with a plurality of slits 100. The slits 100 are preferably transversely-oriented and arranged in rows wherein the slits 100 in adjacent rows are staggered relative to each other so that the slit regions of the absorbent core 42 can expand in the longitudinal direction. In other embodiments, however, such as to create the transverse extensibility in the end regions of the embodiment shown in FIGS. 5 and 6, the absorbent core may have slits, at least some groups of which are longitudinally-oriented so that at least portions of the absorbent core 42 and sanitary napkin 20 can expand in the transverse direction. In addition, as shown in FIG. 12, in preferred versions of this embodiment, a layer of pervious, preferably absorbent material, which is not slit can be provided on top of the slit absorbent core 42 so that liquids will not be able to travel through the slits, and so that the slits will be hidden from the wearer's view when the sanitary napkin is extended. In embodiments where it is not important for the slits to be hidden from view, however, the pervious absorbent material can be positioned beneath the absorbent core 42.

FIG. 13 shows another alternative embodiment of the sanitary napkin of the present invention in which the absorbent core is provided with slits over nearly its entire surface with the exception of a region at each end. In this embodiment, the topsheet 38 is preferably "SELFed" over its entire surface (although it is also possible for the topsheet 38 to only be "SELFed" in selected regions or zones). The backsheet 40 is preferably SELFed in zones that preferably correspond to the extensible intermediate portions 12 and 14 of the sanitary napkin 20. A less extensible absorbent insert 44 may also is placed on top of the absorbent core 42 in this embodiment. Preferably, the unslit regions 102 of the core extend about one inch (about 2.5 cm) inward from each end edge of the core. This type of core is useful particularly when the sanitary napkin is highly flexible to aid in handling the sanitary napkin. Stiffening the ends of the sanitary napkin by not slitting the ends of the absorbent core 42 tends to eliminate any tendency for the ends of the sanitary napkin to fold over on themselves. FIG. 13, thus, also shows that it is possible for some of the components of the absorbent article, such as the topsheet 38, to be provided with complete extensibility across their entire area, while other components, such as the backsheet 40 and absorbent core 42, may only be provided with extensibility in selected regions of their total area. Preferably, the extensible regions of the various components are aligned to form entire regions of the sanitary napkin that are extensible. However, in order to create different effects, or product performance characteristics, it is also possible to provide embodiments in which the extensible portions of the various components of the sanitary napkin are not aligned.

FIG. 14 shows a particularly preferred fastener pattern for the fastener 52 that is used to attach the sanitary napkin 20 to the wearer's panties when the sanitary napkin is provided in the preferred configuration shown. The fastener 52 comprises a plurality of separate spaced-apart zones of fastener material on the garment-facing side 20B of the sanitary napkin 20. In the preferred embodiment shown, the fastener 52 is a pressure sensitive adhesive. Preferably, the adhesive fastener is located on the regions of the backsheet 40 that are not "SELFed" since it is easier to apply adhesive to these smooth surfaces than to the surfaces that have SELFed regions formed therein. More specifically, the fastener 52 is applied in three zones comprising a U-shaped or horseshoe shaped zone in each end region of the sanitary napkin designated 52A and 52B, respectively, and a pair of spaced apart longitudinally-oriented zones designated 52C adjacent each longitudinal side edge 22 of the sanitary napkin in the central region 32 of the sanitary napkin.

Numerous other embodiments of the sanitary napkin of the present invention are possible. In addition, in any of the embodiments described herein, there can be any number of extensible portions and less extensible portions (i.e., there can be a plurality of each). The extensible portions and less extensible portions of various sanitary napkin embodiments can also be arranged in an unlimited number of different patterns.

An absorbent article could also be constructed exactly like the product shown and described in the Tong-ho J. Hsieh EPO patent publication. In addition, an absorbent article could be created in which the less extensible portions of the main body portion are joined together by elastic members. The elastic members would preferably not be continuous elastic members that have been joined to the topsheet or backsheet that merely contract the entire topsheet or backsheet, or a portion thereof in order to induce curvature into the same. Instead, they would preferably be joined to the less extensible portions of the absorbent article, or one of the components thereof, so that the both sides of the absorbent article are equally in tension and the elastic members would serve to bridge the adjacent less extensible portions of the absorbent article and allow them to move and articulate with respect to each other.

The absorbent article of the present invention can also be provided with optional features, such as side flaps (or wings), or other types of side wrapping elements that are joined to the main body portion of the absorbent article (that is, to the absorbent article shown in the drawings) for protecting the wearer's undergarments from soiling and maintaining the absorbent article in place in the wearer's undergarments. Absorbent articles having flaps and/or side wrapping elements of various types are described in U.S. Patent 4,589,876, issued May 20, 1986, to Van Tilburg, U.S. Patent 4,687,478, issued August 18, 1987, to Van Tilburg, U. S. Patent 5,267,992 issued to Van Tilburg on December 7, 1993, U.S. Patent 5,344,416 issued September 6, 1994, to Niihara, U.S. Patent 5,346,486 issued September 13, 1994, to Osborn, et al., and U.S. Patent 5,389,094 entitled "Absorbent Article Having Flaps and Zones of Differential Extensibility" issued February 14, 1995, to Lavash, et al., U.S. Patent Application Serial No. 07/915,133 filed July 23, 1992 (PCT Publication No. WO 94/02096, published February 3, 1994, in the name of Lavash, et al.), U.S. Patent Application Serial No. 08/124,180 filed September 20, 1993 (PCT Publication No. 94/10200, published in the name of Mansfield, et al.), U.S. Patent Application Serial No. 08/277,733 filed July 20, 1994 (PCT Publication No. WO 95/03025, published in the name of Weinberger, et al. on February 2, 1995).

The present invention is also applicable to other types of absorbent articles worn in the crotch region of an undergarment such as pantiliners and incontinence articles. The terms "panty liner" or "pantiliner" refer to absorbent articles that are less bulky than sanitary napkins which are generally worn by women between their menstrual periods. Examples of suitable absorbent articles in the form of pantiliners that can be provided with the extensible and articulating portions described herein are disclosed in U.S. Patent 4,738,676 entitled "Pantiliner" issued to Osborn on April 19, 1988.

The term 'incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults or other incontinent persons. Examples of suitable incontinence articles that can be provided with the extensible and articulating portions described herein are disclosed in U.S. Patent 5,300,054 issued to Feist, et al. on April 5, 1994 and U. S. Patent 5,304,161 issued to Noel, et al. April 19, 1994.

While particular embodiments of the present invention have been illustrated and described, it would be obvious to those skilled in the art that various other changes and modifications can be made without departing from the scope of the invention.

## Claims

1. An absorbent article (20) for wearing in a crotch region of an undergarment, said absorbent article (20) having a longitudinal centerline L, transverse centerline T, a first end edge (24), a second end edge (24), and two spaced apart longitudinal side edges (22), a first portion (6) disposed adjacent said first end edge (24), and a second portion (8) disposed adjacent said second end edge (24), said first portion (6) and said second portion (8) being separated by at least one intermediate portion (12), said absorbent article (20) comprising:
a liquid pervious topsheet (38);
a liquid impervious backsheet (40) joined to said topsheet (38); and
an absorbent core (42) positioned between said topsheet (38) and said backsheet (40),
said absorbent article (20) being **characterized in that** said intermediate portion (12) comprises a flexible and extensible expansion joint located between said first (6) and second (8) portions, said absorbent article (20) also having a first end region (28), a second end region (30), and a central region (32) disposed between said first (28) and second (30) end regions, said absorbent article (20) further comprising a second intermediate portion (14), wherein said intermediate portion (12) is located between said first end region (28) and said central region (32) and said second intermediate portion (14) is located between said central region (32) and said second end region (30), said second intermediate portion (14) comprising a flexible and extensible expansion joint, wherein each of said expansion joints defines a region that extends transversely across said entire absorbent article (20) from one longitudinal side edge (22) to the other longitudinal side edge (22), wherein each of said expansion joints is more extensible than at least some of the remaining portions of said absorbent article (20), said first and second intermediate portions (12, 14) serving as flexible and extensible expansion joints about which the adjacent less extensible portions can articulate to conform to the different regions of the wearer's body.

2. The absorbent article (20) of Claim 1 wherein said absorbent article (20) defines a plane, and said extensible expansion joint is generally extensible in the longitudinal direction in the plane of said absorbent article (20).

3. The absorbent article (20) of Claim 1 wherein at least one of said first (28) and second (30) end regions has a transversely extensible portion therein that is located in a region that surrounds and includes a portion of said longitudinal centerline L.

4. An absorbent article (20) according to any of the preceding claims wherein at least one of said topsheet (38) and said backsheet (40) in at least said expandable region or said expansion joint has a network of at least two distinct regions formed in a portion thereof so that said portion of said at least one of said topsheet (38) and said backsheet (40) in the region of said expansion joint exhibits an elastic-like behavior along at least one predetermined axis.

5. The absorbent article (20) of Claim 1 wherein said first portion (6) is capable of moving laterally relative to said longitudinal centerline L and relative to said second portion (8) so that the longitudinal side edge (22) on one side of said first portion (6) is shifted laterally outboard of the longitudinal side edge (22) of said second portion (8) on the same side of said absorbent article (20).

6. The absorbent article (20) of Claims 1, 2, 3, 4, or 5 wherein said absorbent core (42) is provided with extensibility in at least said intermediate portion (12,14,16,18) by a region of said core (42) having a plurality of slits therein, or by a segmented core structure wherein the segments (42A,42B,42C) of the core are at least partially overlapping.

## Patentansprüche

1. Ein absorbierender Artikel (20) zum Tragen in einem Schrittbereich eines Unterwäschestücks, wobei der genannte absorbierende Artikel (20) eine Längsmittellinie L, eine Quermittellinie T; einen ersten Endrand (24), einen zweiten Endrand (24) und zwei voneinander beabstandete Längsseitenränder (22) umfaßt, wobei ein erster Abschnitt (6) anliegend an den genannten ersten Endrand (24) angeordnet ist, und ein zweiter Abschnitt (8) anliegend an den genannten zweiten Endrand (24) angeordnet ist, wobei der genannte erste Abschnitt (6) und der genannte zweite Anschnitt (8) durch mindestens einen Zwischenabschnitt (12) getrennt sind, wobei der genannte absorbierende Artikel (20) umfaßt:
- ein flüssigkeitsdurchlässiges Deckblatt (38);
- ein flüssigkeitsundurchlässiges Rückenblatt (40), welches mit dem genannten Deckblatt (38) verbunden ist; und
- einen absorbierenden Kern (42); welcher zwischen dem genannten Deckblatt (38) und dem genannten Rückenblatt (40) positioniert ist,
wobei der genannte absorbierende Artikel (20) **dadurch gekennzeichnet ist, daß** der genannte Zwischenabschnitt (12) ein flexibles und verlängerbares Expansionsverbindungsstück aufweist, welches zwischen dem genannten ersten Abschnitt (6) und dem genannten zweiten Abschnitt (8) angeordnet ist, der absorbierende Artikel (20) weist ebenso einen ersten Endbereich (28), einen zweiten Endbereich (30) und einen Mittelbereich (32) auf, welcher zwischen dem genannten ersten Endbereich (28) und dem genannten zweiten Endbereich (30) angeordnet ist, wobei der genannte absorbierende Artikel (20) weiters einen zweiten Zwischenabschnitt (14) umfaßt, wobei der genannte Zwischenabschnitt (12) zwischen dem genannten ersten Endbereich (28) und dem genannten Mittelbereich (32) angeordnet ist und der genannte zweite Zwischenabschnitt (14) zwischen dem genannten Mittelbereich (32) und dem genannten zweiten Endbereich (30) angeordnet ist, der zweite Zwischenabschnitt (14) umfaßt ein flexibles und verlängerbares Expansionsverbindungsstück, wobei jedes der Expansionsverbindungstücke einen Bereich defininiert, welcher sich von einem Längsseitenrand (22) quer über den gesamten absorbierenden Artikel (20) zum anderen Längsseitenrand (22) erstreckt, wobei jedes der Expansionsverbindungsstücke verlängerbar ist, als mindestens einige der verbleibenden Abschnitte des genannten absorbierenden Artikels (20), die genannten ersten und zweiten Zwischenabschnitte dienen als flexible und verlängerbare Expansionsschtücke um die die benachbarten, weniger dehnbaren Bereiche schwenken können um sich verschiedenen Bereichen des Körpers der Trägerin anzupassen.

2. Der absorbierende Artikel (20) nach Anspruch 1, bei welchem der genannte absorbierende Artikel (20) eine Ebene definiert und das genannte verlängerbare Expansionsverbindungsstück in der Ebene des genannten absorbierenden Artikels (20) allgemein in der Längsrichtung verlängerbar ist.

3. Der absorbierende Artikel (20) nach Anspruch 6, bei welchem mindestens einer der genannten ersten (28) und zweiten (30) Endbereiche einen quer verlängerbaren Abschnitt darin aufweist, welcher in einem Bereich angeordnet ist, welcher einen Abschnitt de genannten Längsmittellinie L umgibt und enthält.

4. Ein absorbierende Artikel (20) nach einem der vorhergehenden Ansprüche, bei welchem mindestens eines von dem genannten Deckblatt (38) und dem genannten Rückenblatt (40) in mindestens dem genannten expandierbaren Bereich oder dem genannten Expansionsverbindungsstück ein Netzwerk mit mindestens zwei unterschiedlichen Bereichen aufweist, welche in einem Abschnitt desselben gebildet sind, sodaß der genannte Abschnitt des genannten mindestens einen des genannten Deckblatts (38) und genannten Rückenblatts (40) im Bereich des genannten Expansionsverbindungsstücks ein gummiähnliches Verhalten entlang mindestens einer festgelegten Achse aufweist.

5. Der absorbierende Artikel (20) nach Anspruch 4, bei welchem der genannte erste Abschnitt (6) imstande ist, sich in Bezug auf die genannte Längsmittellinie L und in Bezug auf den genannten zweiten Abschnitt (8) quer zu bewegen, sodaß der Längsseitenrand (22) an einer Seite des genannten ersten Abschnitts (6) außerhalb des genannten Längsseitenrandes (22) des genannten zweiten Abschnitts (8) an der gleichen Seite des genannten absorbierenden Artikel (20) quer verschoben ist.

6. Der absorbierende Artikel (20) nach den Ansprüchen 1, 2, 3, 4, 5 oder 6, bei welchem der genannte absorbierende Kern (42) in mindestens dem genannten Zwischenabschnitt (12, 14, 16, 18) durch einen Bereich des genannten Kerns (42), welcher eine Mehrzahl von Schlitzen darin aufweist, oder durch eine segmentierte Kernstruktur, wobei die Segmente (42A, 42B, 42C) des Kerns mindestens teilweise überlappen, mit Verlängerbarkeit versehen ist.

## Revendications

1. Article absorbant (20) destiné à être porté dans la région d'entrejambe d'un sous-vêtement, ledit article absorbant (20) ayant une ligne médiane longitudinale (L), une ligne médiane transversale (T), un premier bord d'extrémité (24), un deuxième bord d'extrémité (24), et deux bords latéraux longitudinaux (22) écartés l'un de l'autre, une première partie (6) disposée au voisinage dudit premier bord d'extrémité (24), et une deuxième partie (8) disposée au voisinage dudit deuxième bord d'extrémité (24), ladite première partie (6) et ladite deuxième partie (8) étant séparées par au moins une partie intermédiaire (12), ledit article absorbant (20) comprenant:
une feuille de dessus perméable aux liquides (38);
une feuille de fond imperméable aux liquides (40) réunie à ladite feuille de dessus (38); et
une âme absorbante (42) placée entre ladite feuille de dessus (38) et ladite feuille de fond (40),
ledit article absorbant (20) étant **caractérisé en ce que** ladite partie intermédiaire (12) comprend un joint de dilatation flexible et extensible, situé entre ladite première partie (6) et ladite deuxième partie (8), ledit article absorbant (20) ayant également une première région d'extrémité (28), une deuxième région d'extrémité (30), et une région centrale (32) disposée entre ladite première région (28) et ladite deuxième région (30), ledit article absorbant (20) comprenant en outre, une deuxième partie intermédiaire (14), ladite partie intermédiaire (12) étant située entre ladite première région d'extrémité (28) et ladite région centrale (32) et ladite deuxième partie intermédiaire (14) étant située entre ladite région centrale (32) et ladite deuxième région d'extrémité (30), ladite deuxième partie intermédiaire (14) comprenant un joint de dilatation flexible et extensible, chacun desdits joints de dilatation définissant une région qui s'étend transversalement à travers tout ledit article absorbant (20), depuis un bord latéral longitudinal (22) jusqu'à l'autre bord latéral longitudinal (22), chacun desdits joints de dilatation étant plus extensible qu'au moins certaines des parties restantes dudit article absorbant (20), lesdites première et deuxième parties intermédiaires (12, 14) servant de joints de dilatation flexibles et extensibles de façon que les parties adjacentes les moins extensibles puissent s'articuler pour se conformer aux différentes régions du corps de l'utilisatrice.

2. Article absorbant (20) selon la revendication 1, dans lequel ledit article absorbant (20) définit un plan, et ledit joint de dilatation extensible est généralement extensible dans la direction longitudinale, dans le plan dudit article absorbant (20).

3. Article absorbant (20) selon la revendication 1, dans lequel au moins ladite première (28) région d'extrémité ou bien ladite deuxième (30) région d'extrémité comporte une partie extensible dans la direction transversale, qui est située dans une région qui entoure et comprend une partie de ladite ligne médiane longitudinale (L).

4. Article absorbant (20) selon l'une quelconque des revendications précédentes, dans lequel un réseau d'au moins deux régions distinctes est formé dans une partie d'au moins ladite feuille de dessus (38) ou bien ladite feuille de fond (40), dans au moins ladite région dilatable ou ledit joint de dilatation, de telle sorte que ladite partie de ladite feuille de dessus (38) ou bien de ladite feuille de fond (40), dans la région dudit joint de dilatation, présente un comportement de type élastique le long d'au moins un axe prédéterminé.

5. Article absorbant (20) selon la revendication 1, dans lequel ladite première partie (6) est capable de se déplacer dans la direction latérale par rapport à ladite ligne médiane longitudinale (L) et par rapport à ladite deuxième partie (8), si bien que ledit bord latéral longitudinal (22) d'un côté de ladite première partie (6) est décalé vers l'extérieur dans la direction latérale par rapport au bord latéral longitudinal (22) de ladite deuxième partie (8), du même côté dudit article absorbant (20).

6. Article absorbant (20) selon les revendications 1, 2, 3, 4 ou 5, dans lequel une certaine extensibilité est conférée à ladite âme absorbante (42) dans au moins ladite partie intermédiaire (12, 14, 16, 18) grâce au fait qu'une région de ladite âme (42) comporte une pluralité de fentes, ou grâce à une structure d'âme segmentée dans laquelle les segments (42A, 42B, 42C) de l'âme se chevauchent au moins partiellement.
